# EUROPEAN PATENT SPECIFICATION

(11) **EP 1 084 145 B1**
(45) Date of publication and mention of the grant of the patent: **10.08.2005**
(21) Application number: 99923571.6
(22) Date of filing: 11.05.1999
(51) Int. Cl.: C07K 16/00

(54) **ANTIBODIES TO THE ED-B DOMAIN OF FIBRONECTIN, CONJUGATES CONTAINING THEM AND USE THEREOF FOR DIAGNOSIS AND THERAPY OF TUMORS AND DISEASES ASSOCIATED WITH ANGIOGENESIS**
ANTIKÖRPER GEGEN DIE ED-B DOMÄNE VON FIBRONEKTIN, SOLCHE ANTIKÖRPER ENTHALTENDE KONJUGATE, UND DEREN VERWENDUNG ZUR DIAGNOSE UND THERAPIE VON ANGIOGENESE-ASSOZIIERTEN TUMOREN UND KRANKHEITEN
MOLECULES DE LIAISON SPECIFIQUES POUR SCINTIGRAPHIE, CONJUGUES CONTENANT CES MOLECULES ET TRAITEMENT DE L'ANGIOGENESE

(30) Priority: 11.05.1998 US 75338; 28.04.1999 US 300425
(43) Date of publication of application: 21.03.2001
(73) Proprietor: Eidgenössische Technische Hochschule Zürich, 8092 Zürich (CH)
(72) Inventor: NERI, Dario, CH-8049 Zürich (CH); TARLI, Lorenzo, I-53035 Monteriggioni (IT); VITI, Francesca, I-16151 Genova (IT); BIRCHLER, Manfred, CH-8004 Zürich (CH)
(74) Representative: Walton, Seán Malcolm
(86) International application number: PCT/EP1999/003210
(87) International publication number: WO 1999/058570

(56) References cited:
- WO-A-97/45544
- NERI D ET AL: "Targeting by affinity-matured recombinant antibody fragments of an angiogenesis associated fibronectin isoform." NATURE BIOTECHNOLOGY, (1997 NOV) 15 (12) 1271-5. , XP002124779 cited in the application
- NERI, DARIO (1) ET AL: "Affinity reagents against tumour-associated extracellular molecules and newforming vessels." ADVANCED DRUG DELIVERY REVIEWS, (APRIL 6, 1998) VOL. 31, NO. 1-2, PP. 43-52. , XP002124780
- PINI A ET AL: "Design and use of a phage display library. Human antibodies with subnanomolar affinity against a marker of angiogenesis eluted from a two-dimensional gel." JOURNAL OF BIOLOGICAL CHEMISTRY, (1998 AUG 21) 273 (34) 21769-76. , XP002124781
- VITI F ET AL: "Increased binding affinity and valence of recombinant antibody fragments lead to improved targeting of tumoral angiogenesis." CANCER RESEARCH, (1999 JAN 15) 59 (2) 347-52. , XP002124782
- FATTORUSSO R ET AL: "NMR structure of the human oncofoetal fibronectin ED-B domain, a specific marker for angiogenesis." STRUCTURE, (1999 APR 15) 7 (4) 381-90. , XP002124783
- TARLI L ET AL: "A high-affinity human antibody that targets tumoral blood vessels." BLOOD, (1999 JUL 1) 94 (1) 192-8. , XP002124784

## Description

### Field of the Invention

The present invention relates to antibodies with sub-nanomolar affinity specific for a characteristic epitope of the ED-B domain of fibronectin, a marker of angiogenesis. It also relates to the use of radiolabeled high-affinity anti-ED-B antibodies for detecting new-forming blood vessels in vivo and a diagnostic kit comprising said antibody.

Moreover, the invention refers to conjugates comprising the above said antibodies and a suitable photoactive molecule (e.g., a photosensitizer) and to their use in the detection and/or coagulation of new blood vessels.

### Background of the Invention

Tumours cannot grow beyond a certain mass without the formation of new blood vessels (angiogenesis), and a correlation between microvessel density and tumour invasiveness has been reported for a number of tumours (Folkman (1995). Nature Med., 1, 27-31). Moreover, angiogenesis underlies the majority of ocular disorders which result in loss of vision [Lee et al., *Surv. Ophthalmol*. 43, 245-269 (1998); Friedlander, M. *et al., Proc. Nati. Acad. Sci. U.S.A.* 93, 9764-9769 (1996).]. Molecules capable of selectively targeting markers of angiogenesis would create clinical opportunities for the diagnosis and therapy of tumours and other diseases characterised by vascular proliferation, such as diabetic retinopathy and age-related macular degeneration. Markers of angiogenesis are expressed in the majority of aggressive solid tumours and should be readily accessible to specific binders injected intravenously (Pasqualini et al. (1997). Nature Biotechnol., 15, 542-546; Neri et al. (1997), Nature Biotechnol., 15 1271-1275). Targeted occlusion of the neovasculature may result in tumour infarction and collapse (O'Reilly et al. (1996). Nature Med., 2, 689-692; Huang et al. (1997). Science, 275, 547-550).

The ED-B domain of fibronectin, a sequence of 91 aminoacids identical in mouse, rat and human, which is inserted by alternative splicing into the fibronectin molecule, specifically accumulates around neo-vascular structures (Castellani et al. (1994). Int. J. Cancer 59, 612-618) and could represent a target for molecular intervention. Indeed, we have recently shown with fluorescent techniques that anti-ED-B single-chain Fv antibody fragments (scFv) accumulate selectively in tumoural blood vessels of tumour-bearing mice, and that antibody affinity appears to dictate targeting performance (Neri et al. (1997). Nature Biotechnol., 15 1271-1275; International Patent Application No. PCT/GB97/01412, based on GB96/10967.3). Tumour targeting was evaluated 24 hours after injection, or at later time points.

Various attempts are known in the art to raise antibodies against the ED-B-domain in order to use them for tumour targeting.

Peters et al. (Cell Adhesion and Communication 1995, 3: 67-89) disclose polyclonal antibodies raised to antigens containing no FN sequence other than the intact ED-B domain and show that they bind specifically and directly to this domain.

However, the reagents of Peters et al. suffer from a series of drawbacks:- the antisera of Peters et al. recognise ED-B(+)-FN only after treatment with N-glycanase. This makes these reagents unsuitable for applications such as tumour targeting, imaging and therapy, as deglycosylation cannot be performed in vivo.

The authors acknowledge themselves that their antibodies do not recognise full-length ED-B(+)-FN produced by mammalian cells. They also acknowledge that it had been impossible to produce monoclonal antibodies specific for the ED-B domain of fibronectin, even though antibodies against other domains of fibronectin (such as ED-A) had been produced. It is well-known in the art that polyclonal antisera are unacceptable for above mentioned applications.

Even after years of intense research in this field, monoclonal antibodies recognising the ED-B domain of fibronectin without treatment with N-glycanase could be produced only using phage display techniques as applied in the present invention.

Zang et al. (Matrix Biology 1994, 14: 623-633) disclose a polyclonal antiserum raised against the canine ED-B domain. The authors do expect a cross-reactivity to human ED-B(+)-FN, although this was not tested. However, the authors acknowledge the difficulty to produce monoclonal antibodies directly recognising the ED-B domain of fibronectin (page 631). The antiserum recognises ED-B(+)-FN in Western blot only after treatment with N-glycanase. As mentioned before, glycanase treatment renders these reagents unsuitable for applications according to the present invention.

Recognition of ED-B(+)-FN in ELISA proceeds without the need of deglycosylation but only on cartilage extracted with a denaturing agent (4M Urea) and captured on plastic using gelatin. The authors comment that "the binding of the FN molecule to the gelatine bound on the plastic surface of the ELISA plate may somehow expose the epitopes sufllcienby for recognition by the antiserum". Since for in vivo applications FN cannot be denatured and gelatin bound, the monoclonal binders of the present invention offer distinct advantages.

Neri & Zardi (Advanced Drug Delivery Reviews 1998, 31:43-52) discuss tumour targeting using extracellular matrix components of tumour stroma, such as fibronectin and tenascin, and the possibility of using human recombinant antibodies in tumour targeting.

The Japanese patents JP02076598 and JP04169195 refer to anti-ED-B antibodies. It is not clear from these documents if monoclonal anti ED-B antibodies are described. Moreover, it seems impossible that a single antibody (such as the antibody described in JP02076598) has "an antigen determinant in aminoacid sequence of formulae (1), (2) or (3):
- (1) EGIPIFEDFVDSSVGY
- (2) YTVTGLEPGIDYDIS
- (3) NGGESAPTTLTQQT
on the basis of the following evidence:
i) A monoclonal antibody should recognise a well-defined epitope.
ii) The three-dimensional structure of the ED-B domain of fibronectin has been determined by NMR spectroscopy. Segments (1), (2) and (3) lie on opposite faces of the ED-B structure, and cannot be bound simultaneously by one monoclonal antibody.

Furthermore, in order to demonstrate the usefulness of the antibodies localisation in tumours should be demonstrated, as well as evidence of staining of ED-B(+)-FN structures in biological samples without treatment with structure-disrupting reagents.

The BC1 antibody described by Carnemolla et al. 1992, J. Biol. Chem. 267, 24689-24692, recognises an epitope on domain 7 of FN, but not on the ED-B domain, which is cryptic in the presence of the ED-B domain of fibronectin. It is strictly human-specific. Therefore, the BC1 antibody and the antibodies of the present invention show different reactivity. Furthermore, the BC1 antibody recognises domain 7 alone, and domain 7-8 of fibronectin in the absence of the ED-B domain (Carnemolla et al. 1992, J. Biol. Chem. 267, 24689-24692). Such epitopes could be produced in vivo by proteolytic degradation of FN molecules. The advantage of the reagents according to the present invention is that they can localise on FN molecules or fragments only if they contain the ED-B domain.

For the diagnosis of cancer, and more specifically for imaging primary and secondary tumour lesions, immunoscintigraphy is one of the techniques of choice.

In this methodology, patients are imaged with a suitable device (e.g., a gamma camera), after having been injected with radiolabeled compound (e.g., a radionuclide linked to a suitable vehicle). For scintigraphic applications, short-lived gamma emitters such as technetium-99m, iodine-123 or indium-111 are typically used, in order to minimise exposure of the patient to ionising radiations.

The most frequently used radionuclide in Nuclear Medicine Departments is technetium-99m (99mTc), a gamma emitter with half-life of six hours. Patients injected with 99mTc-based radiopharmaceuticals can typically be imaged up to 12-24 hours after injections; however, accumulation of the nuclide on the lesion of interest at earlier time points is desirable.

Furthermore, if antibodies capable of rapid and selective localisation on newly-formed blood vessels were available, researchers would be stimulated to search for other suitable molecules to conjugate to antibodies, in order to achieve diagnostic and/or therapeutic benefit.

### Summary of the Invention

Considering the need of nuclear medicine for radiopharmaceuticals capable of localising tumour lesions few hours after injection, and the information that antibody affinity appears to influence its performance in targeting of angiogenesis, it is an object of the present invention to produce antibodies specific for the ED-B domain of fibronectin with a dissociation constant of less than 1 x 10⁻⁹ M (for a review on the definitions and measurements of antibody-antigen affinity, see Neri et al. (1996). Trends in Biotechnol. 14, 465-470). A further object of the present invention is to provide radiolabeled antibodies in suitable format, directed against the ED-B domain of fibronectin, that detect tumour lesions already few hours after injection.

In one aspect of the invention these objects are achieved by an antibody with specific affinity for a characteristic epitope of the ED-B domain of fibronectin and with improved affinity to said ED-B epitope.

In a further aspect of the present invention the above described antibody is used in a method of rapid targeting markers of angiogenesis.

Another aspect of the present invention is a diagnostic kit comprising said antibody and one or more reagents for detecting angiogenesis.

Still a further aspect of the present invention is the use of said antibody in the manufacture of a diagnostic agent or a medicament for diagnosis and therapy of tumours and diseases which are characterized by vascular proliferation.

Finally, an important aspect of the invention is represented by conjugates comprising said antibodies and a suitable photoactive molecules (e.g. a judiciously chosen photosensitizer), and their use for the selective light-mediated occlusion of new blood vessels.

### Terminology

Throughout the application several technical expressions are used for which the following definitions apply.
- antibody
   This describes an immunoglobulin whether natural or partly or wholly synthetically produced. The term also covers any polypeptide or protein having a binding domain which is, or is homologous to, an antibody binding domain. These can be derived from natural sources, or they may be partly or wholly synthetically produced. Examples of antibodies are the immunoglobulin isotypes and their isotypic subclasses; fragments which comprise an antigen binding domain such as Fab, scFv, Fv, dAb, Fd; and diabodies. It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB 2188638A or EP-A-239400. A hybridoma or other cell producing an antibody may be subject to genetic mutation or other changes, which may or may not alter the binding specificity of antibodies produced. As antibodies can be modified in a number of ways, the term "antibody" should be construed as covering any specific binding member or substance having a binding domain with the required specificity. Thus, this term covers antibody fragments, derivatives, functional equivalents and homologues of antibodies, including any polypeptide comprising an immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023. It has been shown that fragments of a whole antibody can perform the function of binding antigens. Examples of binding fragments are (I) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward et al. (1989) Nature, 9, 341, 544-546.) which consists of a VH domain; (v) isolated CDR regions; (vi) F(ab')2 fragments, a bivalent fragment comprising two linked Fab fragments; (vii) single chain Fv molecules (scFv), wherein a VH domain and a VL domain are linked by a polypeptide linker which allows the two domains to associate to form an antigen binding site (Bird et al. (1988) Science, 242, 423-426.; Huston et al. (1988) Proc. Natl. Acad. Sci. U.S.A., 85, 5879-83.); (viii) bispecific single chain FV dimers (PCT/US92/09965) and (ix) "diabodies", multivalent or multispecific fragments constructed by gene fusion (WO94/13804; Holliger et al. (1993) Proc. Natl. Acad. Sci. U.S.A., 90, 6444-6448). Diabodies are multimers of polypeptides, each polypeptide comprising a first domain comprising a binding region of an immunoglobulin light chain and a second domain comprising a binding region of an immunoglobulin heavy chain, the two domains being linked (e.g. by a petide linker) but unable to associate with each other to form an antigen binding site: antigen binding sites are formed by the association of the first domain of one polypeptide within the multimer with the second domain of another polypeptide within the multimer (WO94/13804). Where bispecific antibodies are to be used, these may be conventional bispecific antibodies, which can be manufactured in a variety of ways Holliger and Winter (1993), Curr. Opin. Biotech., 4, 446-449), e.g. prepared chemically or from hybrid hybridomas, or may be any of the bispecific antibody fragments mentioned above. It may be preferable to use scFv dimers or diabodies rather than whole antibodies. Diabodies and scFv can be constructed without an Fc region, using only variable domains, potentially reducing the effects of anti-idiotypic reaction. Other forms of bispecific antibodies include the single-chain CRAbs described by Neri et al. ((1995) J. Mol. Biol., 246, 367-373).
- complementarity-determining regions
   Traditionally, complementarity-deterining regions (CDRs) of antibody variable domains have been identified as those hypervariable antibody sequences, containing residues essential for specific antigen recognition. In this document, we refer to the CDR definition and numbering of Chothia and Lesk (1987) J. Mol. Biol., 196, 901-917.
- functionally equivalent variant form
   This refers to a molecule (the variant) which although having structural differences to another molecule (the parent) retains some significant homology and also at least some of the biological function of the parent molecule, e.g. the ability to bind a particular antigen or epitope. Variants may be in the form of fragments, derivatives or mutants. A variant, derivative or mutant may be obtained by modification of the parent molecule by the addition, deletion, substitution or insertion of one or more aminoacids, or by the linkage of another molecule. These changes may be made at the nucleotide or protein level. For example, the encoded polypeptide may be a Fab fragment which is then linked to an Fc tail from another source. Alternatively, a marker such as an enzyme, fluorescein, etc, may be linked. For example, a functionally equivalent variant form of an antibody "A" against a characteristic epitope of the ED-B domain of fibronectin could be an antibody "B" with different sequence of the complementarity determining regions, but recognising the same epitope of antibody "A".
   We have isolated recombinant antibodies in scFv format from an antibody phage display library, specific for the ED-B domain of fibronectin, and recognising ED-B(+)-fibronectin in tissue sections. One of these antibodies, E1, has been affinity matured to produce antibodies H10 and L19, with improved affinity. Antibody L19 has a dissociation constant for the ED-B domain of fibronectin in the sub-nanomolar concentration range.
   The high-affinity antibody L19 and D1.3 (an antibody specific for an irrelevant antigen, hen egg lysozyme) were radiolabeled and injected in tumour-bearing mice. Tumour, blood and organ biodistributions were obtained at different time points, and expressed as percent of the injected dose per gram of tissue (%ID/g).
   Already 3 hours after injection, the %ID/g (tumour) was better than the %ID/g (blood) for L19, but not for the negative control D1.3. The tumour : blood ratios increased at longer time points. This suggests that the high-affinity antibody L19 may be a useful tumour targeting agent, for example for immunoscintigraphic detection of angiogenesis.
- photosensitizer (or photosensitiser)
   A photosensitiser could be defined as a molecule which, upon irradiation and in the presence of water and/or oxygen, will generate toxic molecular species (e.g., singlet oxygen) capable of reacting with biomolecules, therefore potentially causing damage to biological targets such as cells, tissues and body fluids.
   Photosensitisers are particularly useful when they absorb at wavelengths above 600 nm. In fact, light penetration in tissues and body fluids is maximal in the 600 - 900 nm range [Wan et al. (1981) Photochem. Photobiol. 34, 679-681).
   The targeted delivery of photosensitisers followed by irradiation is an attractive avenue for the therapy of angiogenesis-related diseases [Yarmush, M.L. *et al*.
   Antibody targeted photolysis. *Crit. Rev. Therap. Drug Carrier Systems* 10, 197-252 (1993); Rowe, P.M. *Lancet* 351, 1496 (1998); Levy, J. *Trends Biotechnol*. 13, 14-18 (1995)), particularly for the selective ablation of ocular neovasculature.
   Available therapeutic modalities such as laser photocoagulation, either directly or after administration of photosensitising agents, are limited by a lack of selectivity and typically result in the damage of healthy tissues and vessels [Macular Photocoagulation Study Group, *Arch. Ophtalm.* 112, 480-488 (1994); Haimovici, R. *et al*., *Curr. Eye Res.* 16, 83-90 (1997); Schmidt-Erfurth, U. *et al*.; *Graefes Arch. Clin. Exp. Ophthalmol*. 236, 365-374 (1998).].
   On the basis of the arguments presented above, one can see that it would be extremely important to discover ways to improve the selectivity and specificity of photosensitisers, for example by conjugating them to a suitable carrier molecule. It is likely that the development of good-quality carrier molecules will not be a trivial task. Moreover, it is likely that not all photosensitisers will lend themselves to be "vehicled" in vivo to the site of interest. Factors such as photosensitiser chemical structure, solubility, lipophilicity, stickiness and potency are likely to crucially influence the "targetability" and efficacy of photosensitisers' conjugates.
   Here we show that the high-affinity L19 antibody, specific for the ED-B domain of fibronectin selectively localises to newly formed blood vessels in a rabbit model of ocular angiogenesis upon systemic administration. The L19 antibody, chemically coupled to the photosensitising agent tin (IV) chlorin e₆ and irradiated with red light, mediated the selective occlusion of ocular neovasculature and promoted apoptosis of the corresponding endothelial cells. These results demonstrate that new ocular blood vessels can be distinguished immunochemically from pre-existing ones in vivo, and strongly suggest that targeted delivery of photosensitisers followed by irradiation may be effective in treating blinding eye diseases and possibly other pathologies associated with angiogenesis.

### Brief Description of the Drawings

Embodiments of the present invention are illustrated by the following figures, wherein
Fig. 1 shows a designed antibody phage library;
Fig. 2 shows 2D gels and Western blotting of a lysate of human melanoma COLO-38 cells;
Fig. 3A, 3B, 3C show immunohistochemical experiments of glioblastoma multiforme
Fig. 4 shows an analysis of the stability of antibody-(ED-B) complexes.
Fig. 5 shows biodistribution of tumour bearing mice injected with radiolabelled antibody fragments.
Fig. 6 shows amino acid sequence of L19;
Figures 7A and 7B show rabbit eyes with implanted pellet;
Figure 8 shows immunohistochemistry of rabbit cornea sections.
Figure 9 shows the immunohistochemistry of sections of ocular structures of rabbits (cornea, iris and conjunctiva) using a red alkaline phoshatase substrate and hematoxylin.
Figure 10 shows the localisation of fluorescently-labeled antibodies in ocular neovasculature.
Figure 11 shows the macroscopic appearance of the eyes of rabbits injected with proteins coupled to photosensitizers, before and after irradiation.
Figure 12 shows the microscopic analysis of sections of ocular structures of rabbits injected with proteins coupled to photosensitizers and irradiated with red light.

### Detailed Description of the Drawings

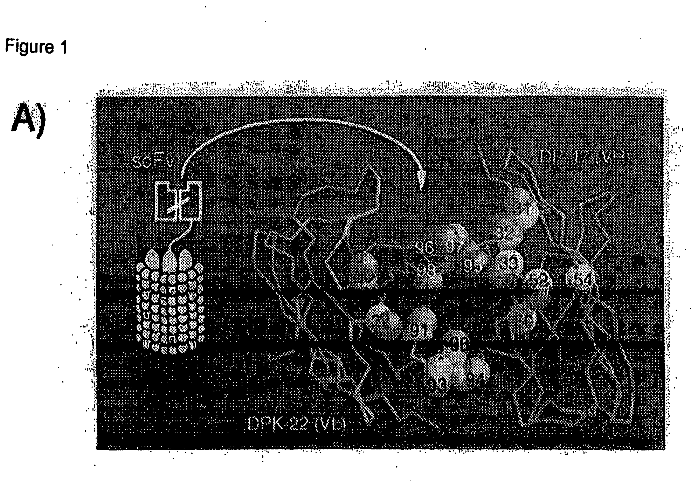

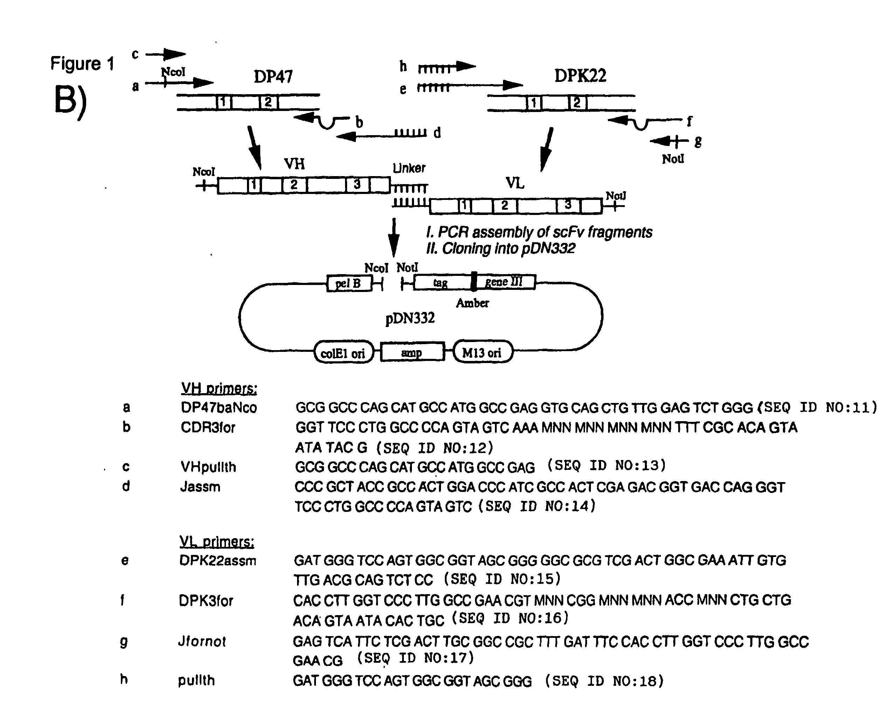

Figure 1 shows:
   Designed antibody phage library. (a) Antibody fragments are displayed on phage as pIII fusion, as schematically depicted. In the antibody binding site (antigen's eye view), the Vk CDRs backbone is in yellow, the VH CDR backbone is in blue. Residues subject to random mutation are Vk CDR3 positions 91, 93, 94 and 96 (yellow), and VH CDR3 positions 95, 96, 97, and 98 (blue). The Cb atoms of these side chains are shown in darker colours. Also shown (in grey), are the residues of CDR1 and CDR2, which can be mutated to improve antibody affinity. Using the program RasMol (http://www.chemistry.ucsc.edu/wipketteaching/rasmol.html), the structure of the scFv were modeled from pdb file 1igm (Brookhaven Protein Data Bank; http://www2.ebi.ac.uk/pcserv/pdbdb.htm). (b) PCR amplification and library cloning strategy. The DP47 and DPK22 germline templates were modified (see text) to generate mutations in the CDR3 regions. Genes are indicated as rectangles, and CDRs as numbered boxes within the rectangle. The VH and the VL segments were then assembled and cloned in pDN332 phagemid vector. Primers (from SEQ ID NO:11 to SEQ ID NO:18) used in the amplification and assembly are listed at the bottom.

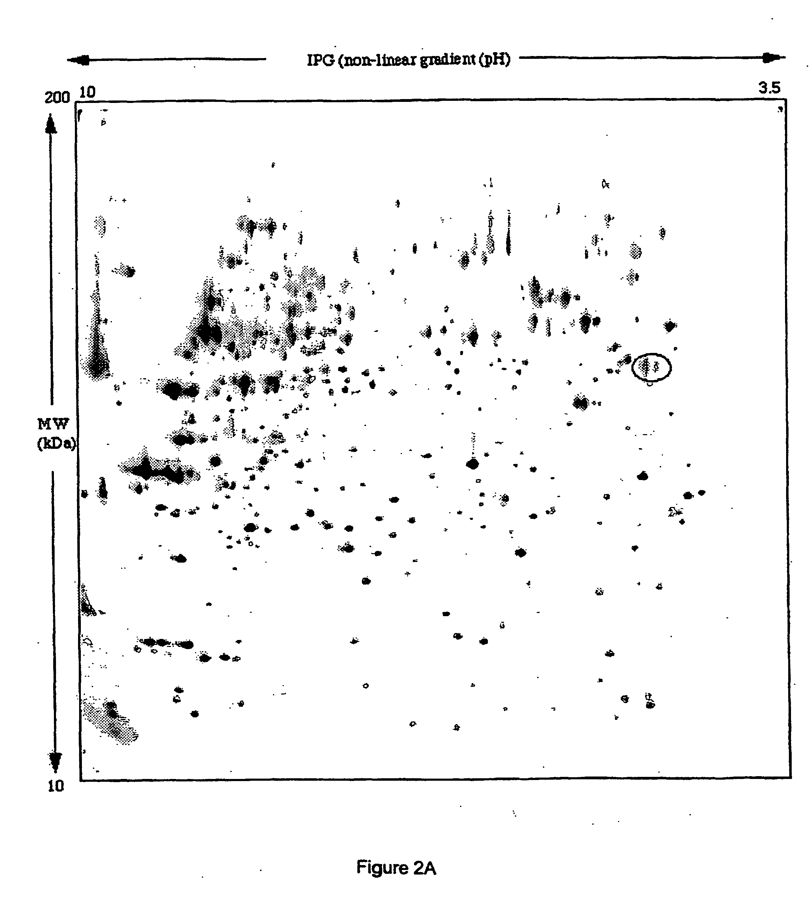

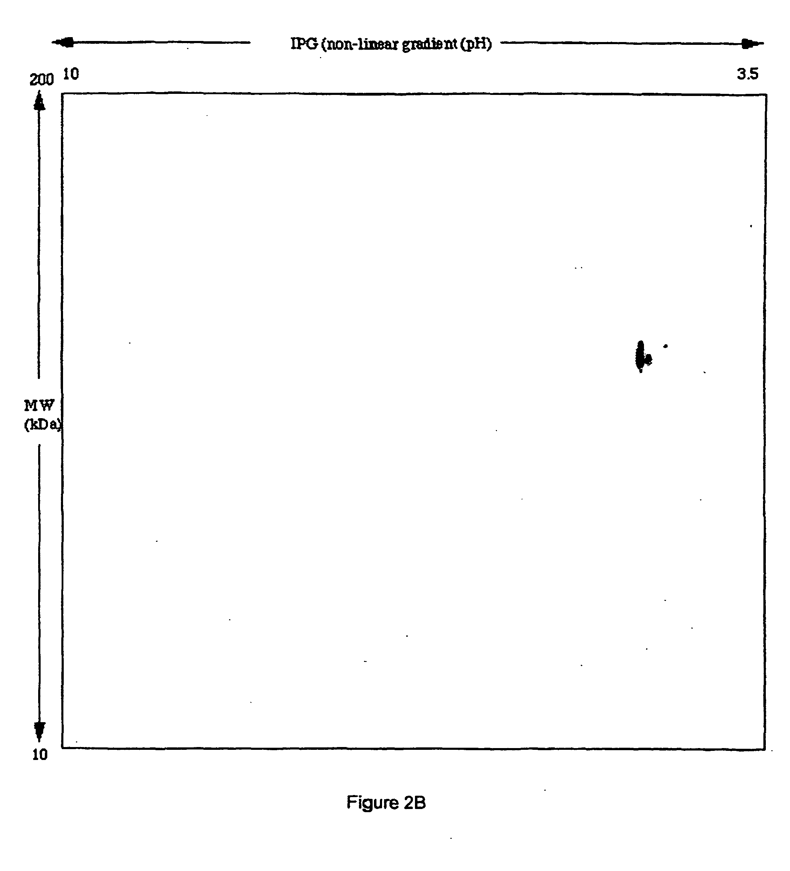

Figure 2) shows
   2D gels and western blotting (a) Silver-staining of the 2D-PAGE of a lysate of human melanoma COLO-38 cells, to which recombinant ED-B-containing 7B89 had been added. The two 7B89 spots (circle) are due to partial proteolysis of the His-tag used for protein purification. (b) Immunoblot of a gel, identical to the one of Fig. 2a, using the anti-ED-B E1 (Table 1) and the M2 anti-FLAG antibodies as detecting reagent. Only the 7B89 spots are detected, confirming the specificity of the recombinant antibody isolated from a gel spot.

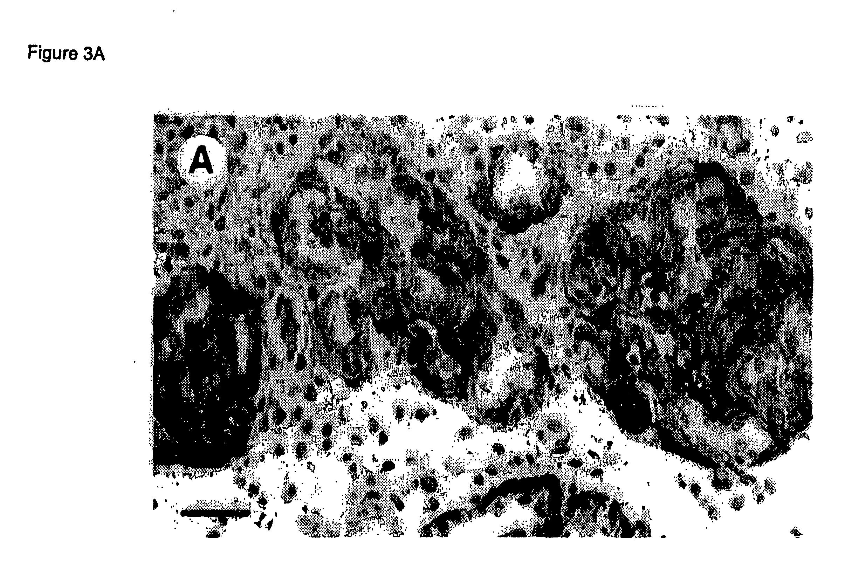

Figures 3A-3C show:
   Immunohistochemical experiments on serial sections of glioblastoma multiforme showing the typical glomerulus-like vascular structures stained using scFvs E1 (A), A2 (B) and G4 (C). Scale bars: 20 µm.

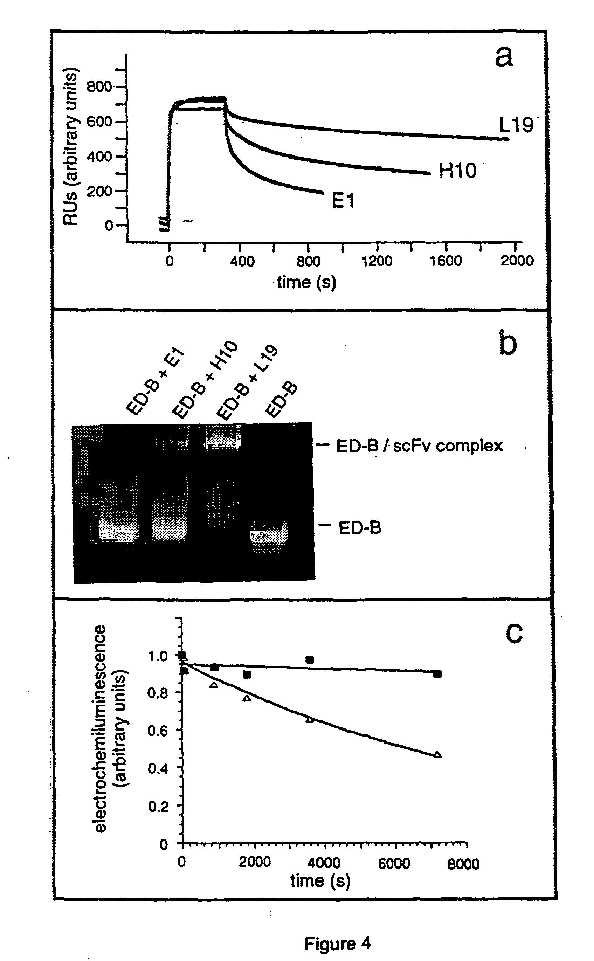

Figure 4) shows:
   Stability of antibody-(ED-B) complexes. Analysis of the binding of scFvs E1, H10 and L19 to the ED-B domain of fibronectin. (a) BIAcore sensograms, showing the improved dissociation profiles obtained upon antibody affinity-maturation. (b) Native gel electrophoretic analysis of scFv-(ED-B) complexes. Only the high-affinity antibody L19 can form a stable complex with the fluorescently labeled antigen. Fluorescence detection was performed as described (Neri et al. (1996) BioTechniques, 20, 708-712).
   (c) Competition of the scFv-(ED-B-biotin) complex with a 100-fold molar excess of unbiotinylated ED-B, monitored by electrochemiluminescence using an Origen apparatus. A long half-life for the L19-(ED-B) complex can be observed. Black squares: L19; Open triangles: H10.

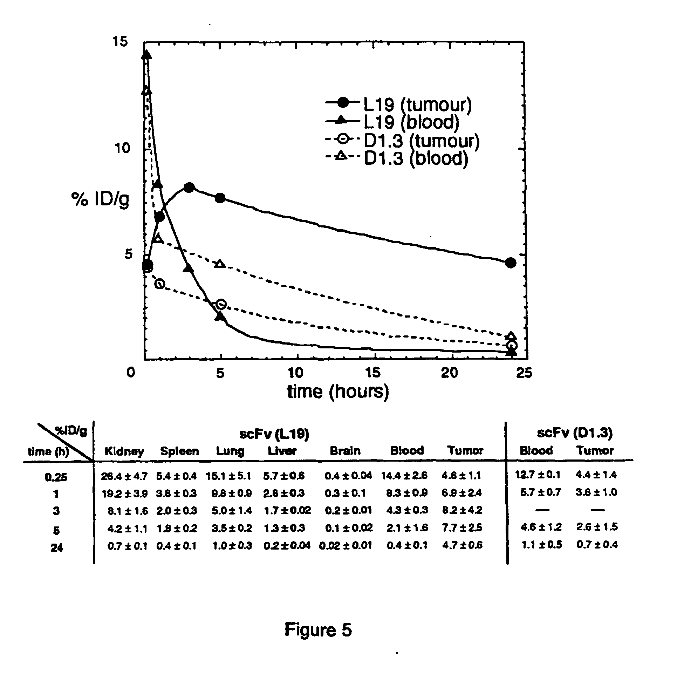

Figure 5) shows:
   Biodistributions of tumour bearing mice injected with radiolabeled antibody fragments.
   Tumour and blood biodistributions, expressed as percent injected dose per gram, are plotted versus time. Relevant organ biodistributions is also reported.

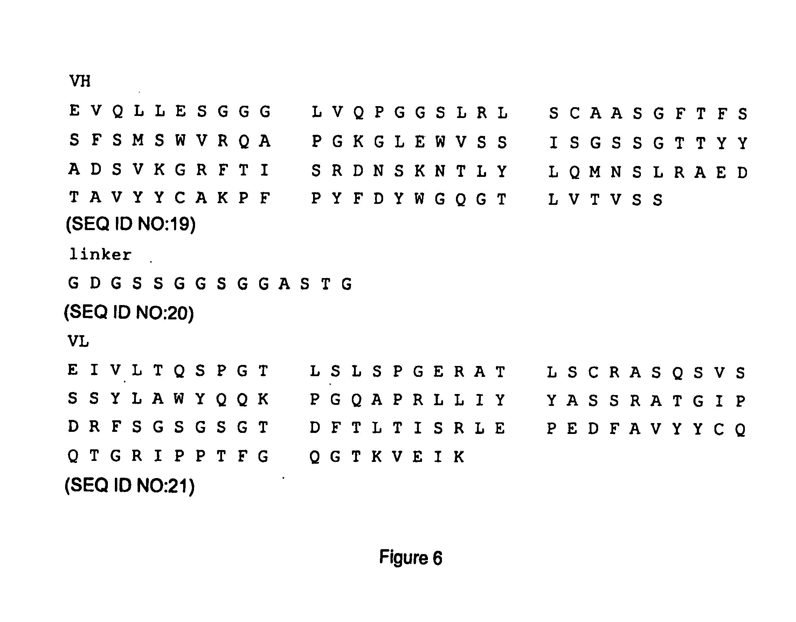

Figure 6) shows the amino acid sequence of antibody L19 comprising the heavy chain (VH), the linker and the light chain (VL).

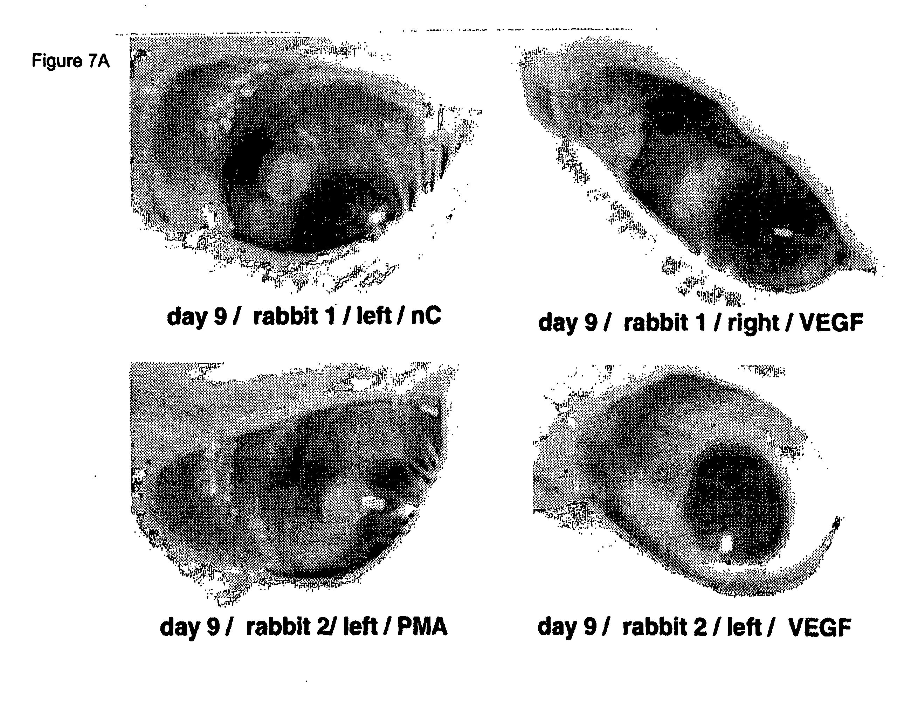

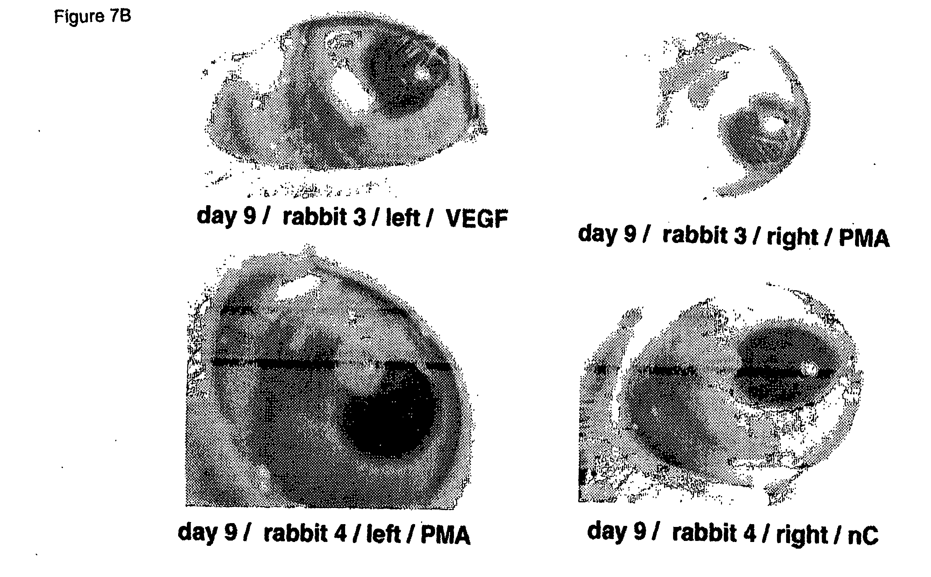

Figures 7A and 7B) show rabbit eyes with implanted polymer pellets soaked with angiogenic substances.

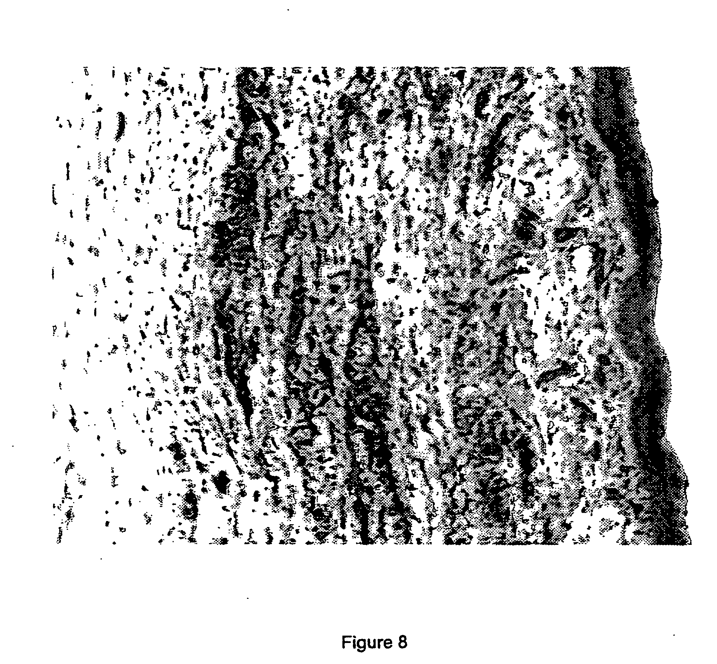

Figure 8) shows immunohistochemistry of sections of rabbit cornea with new-forming blood vessels, stained with the L19 antibody.

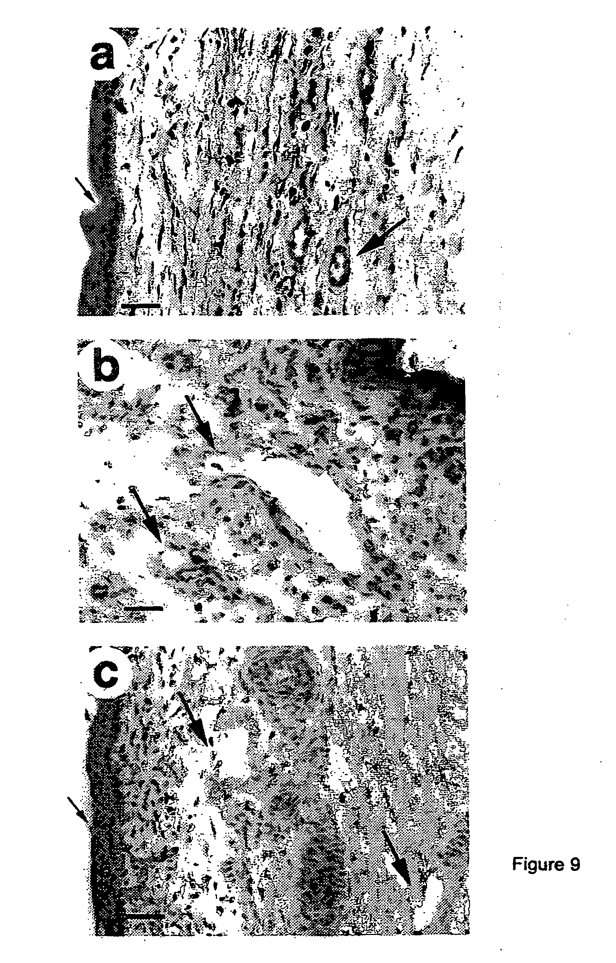

Figure 9) shows immunohistochemical studies of ocular structures using the L19 antibody. A specific red staining is observed around neovascular structures in the cornea (a), but not around blood vessels in the iris (b) and in the conjunctiva (c). Small arrows: corneal epithelium. Relevant blood vessels are indicated with large arrows. Scale bars: 50 µm

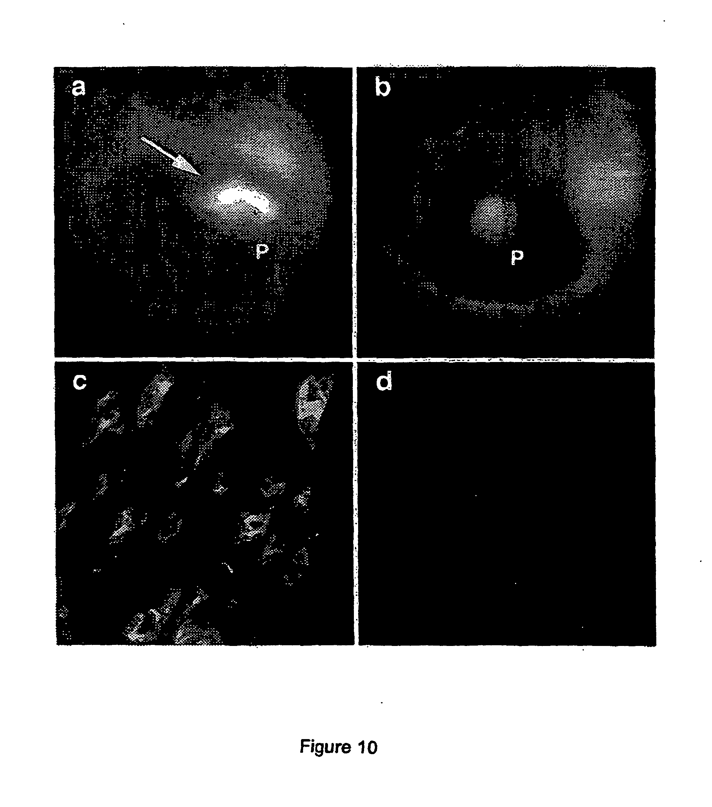

Figure 10) shows immunophotodetection of fluorescently labeled antibodies targeting ocular angiogenesis. A strongly fluorescent corneal neovascularisation (indicated by an arrow) is observed in rabbits injected with the antibody conjugate L19-Cy5 (a), specific for the ED-B domain of FN, but not with the antibody HyHEL-10-Cy5 (b). Immunofluorescence microscopy on cornea sections confirmed that L19-Cy5 (c), but not HyHEL-10-Cy5 (d) localises around neovascular structures in the cornea. Images (a,b) were acquired 8 h after antibody injection; (c, d) were obtained using cornea sections isolated from rabbits 24 h after antibody injection. P, pellet.

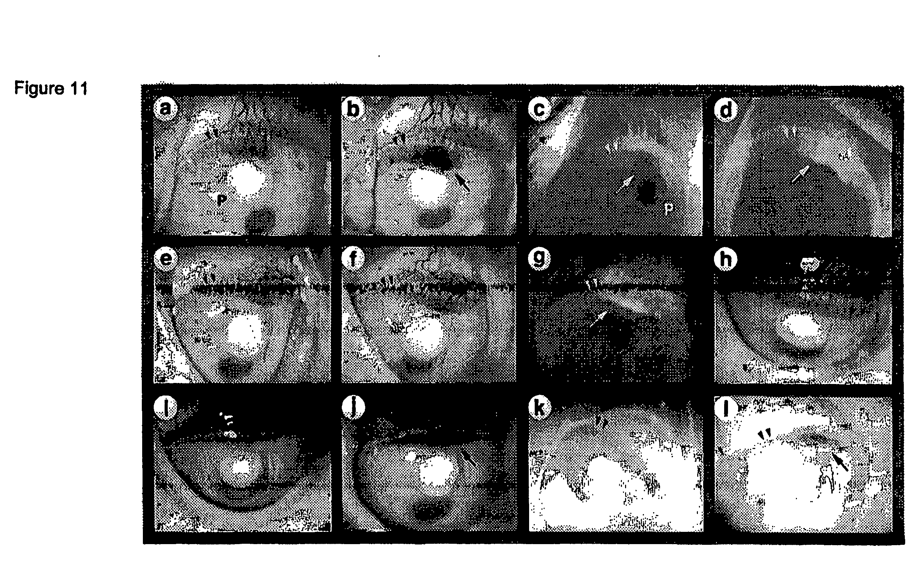

Figure 11) shows macroscopic images of eyes of rabbits treated with photosensitiser conjugates. Eye of rabbit injected with L19-PS before (a) and 16 h after irradiation with red light (b). The arrow indicates coagulated neovasculature, which is confirmed as a hypofluorescent area in the Cy5 fluoroangiogram of panel (c) 16 h after irradiation. Note that no coagulation is observed in other vascular structures, for example in the dilated conjuctival vessels. For comparison, a Cy5 fluoroangiogram with hyperfluorescence of leaky vessels, and the corresponding colour photograph of untreated rabbit eye are shown in (d) and (h). Pictures (e,f,g) are analogous to (a,b,c), but correspond to a rabbit injected with ovalbumin-PS and irradiated with red light. No coagulation can be observed, and the angiogram reveals hyperfluorescence of leaky vessels. The eyes of rabbits with early-stage angiogenesis and injected with L19-PS are shown in (i-I). Images before (i) and 16 h after irradiation with red light (j) reveal extensive and selective light-induced intravascular coagulation (arrow). Vessel occlusion (arrow) is particularly evident in the irradiated eye (I) of a rabbit immediately after euthanasia, but cannot be detected in the non-irradiated eye (k) of the same rabbit. P, pellet. Arrowheads indicate the corneo-scleral junction (limbus). In all figures, dilated pre-existing conjunctival vessels are visible above the limbus, whereas growth of corneal neovascularisation can be observed from the limbus towards the pellet (P).

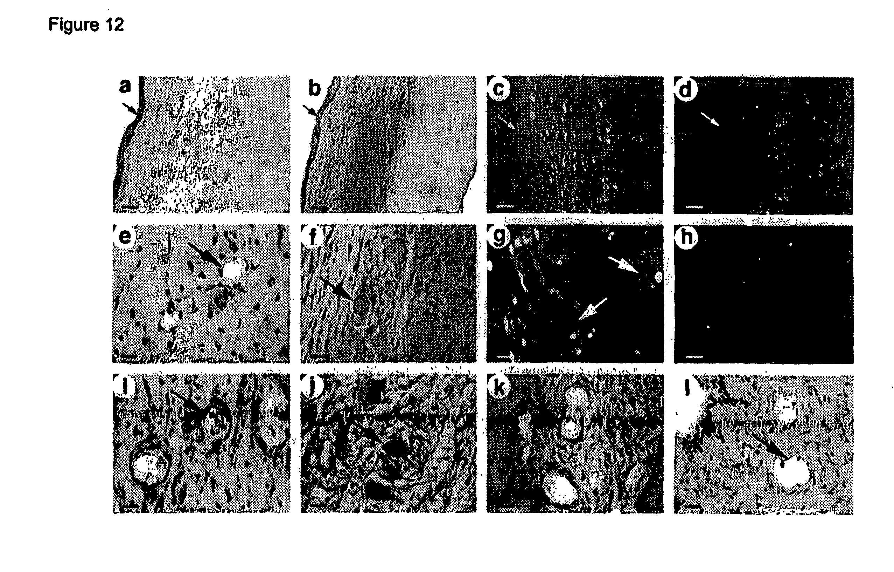

Figure 12) shows microscopic analysis of selective blood vessel occlusion. H/E sections of corneas (a,e,b,f: non-fixed; i,j: paraformaldehyde fixed) of rabbits injected with ovalbumin-PS (a,e,i) or L19-PS (b,f,j) and irradiated. Large arrows indicate representative non damaged (e,i) or completely occluded (f,j) blood vessels. In contrast to the selective occlusion of corneal neovasculature and restricted perivascular damage (eosinophilia) mediated by L19-PS after irradiation (b,f,j), vessels in the conjunctiva (k) and iris (l) do not show sign of damage in the same rabbit. Fluorescent TUNEL assay indicates the different number of apoptotic cells in sections of irradiated rabbits injected with L19-PS (c,g) or with ovalbumin-PS (d,h). Large arrows indicate some relevant vascular structures. Small arrows indicate corneal epithelium. Scale bars: 100 µm (a-d) and 25 µm (e-I)
   The invention is more closely described by the following examples.

### Example 1

### Isolation of human scFv antibody fragments specific for the ED-B domain of fibronectin from a antibody phage-display library

A human antibody library was cloned using VH (DP47; Tomlinson et al. (1992). J. Mol. Biol., 227 , 776-798.) and Vk (DPK22; Cox et al. (1994). Eur. J. Immunol., 24, 827-836) germline genes (see Figure 1 for the cloning and amplification strategy). The VH component of the library was created using partially degenerated primers (Figure 1) (from SEQ ID NO:11 to SEQ ID NO:18) in a PCR-based method to introduce random mutations at positions 95-98 in CDR3. The VL component of the library was generated in the same manner, by the introduction of random mutations at positions 91, 93, 94 and 96 of CDR3. PCR reactions were performed as described (Marks et al. (1991). J. Mol. Biol., 222, 581-597). VH-VL scFv fragments were constructed by PCR assembly (Figure 1; Clackson et al. (1991). Nature , 352, 624-628), from gel-purified VH and VL segments. 30µg of purified VH-VL scFv fragments were double digested with 300 units each of Ncol and Notl, then ligated into 15 µg of Not1/Nco1 digested pDN332 phagemid vector. pDN332 is a derivative of phagemid pHEN1 (Hoogenboom et al. (1991). Nucl. Acids Res., 19, 4133-4137), in which the sequence between the Not1 site and the amber codon preceding the gene III has been replaced by the following sequence, coding for the D3SD3-FLAG-His6 tag (Neri et al. (1996). Nature Biotechnology, 14, 385-390):

Transformations into TG1 E.coli strain were performed according to Marks et al. (1991. J. Mol. Biol., 222, 581-597) and phages were prepared according to standard protocols (Nissim et al. (1991). J. Mol. Biol., 222, 581-597). Five clones were selected at random and sequenced to check for the absence of pervasive contamination.

Recombinant fibronectin fragments ED-B and 7B89, containing one and four type III homology repeats respectively, were expressed from pQE12-based expression vectors (Qiagen, Chatsworth, CA, USA) as described (Carnemolla et al. (1996). Int. J. Cancer, 68, 397-405).

Selections against recombinant ED-B domain of fibronectin (Carnemolla et al. (1996). Int. J. Cancer, 68, 397-405, Zardi et al. (1987). EMBO J., 6, 2337-2342) were performed at 10 nM concentration using the antigen biotinylated with biotin disulfide N-hydroxysuccinimide ester (reagent B-4531; Sigma, Buchs, Switzerland; 10) and eluted from a 2D gel, and streptavidin-coated Dynabeads capture (Dynal, Oslo, Norway). 1013 phages were used for each round of panning, in 1 ml reaction. Phages were incubated with antigen in 2% milk/PBS (MPBS) for 10 minutes. To this solution, 100 µl Dynabeads (10 mg/ml; Dynal, Oslo, Norway), preblocked in MPBS, were added. After 5 min. mixing, the beads were magnetically separated from solution and washed seven times with PBS-0.1% Tween-20 (PBST) and three times with PBS. Elution was carried out by incubation for 2 min. with 500 µl 50 mM dithiothreitol (DTT), to reduce the disulfide bridge between antigen and biotin. Beads were captured again, and the resulting solution was used to infect exponentially growing TG1 E.coli cells. After three rounds of panning, the eluted phage was used to infect exponentially-growing HB2151 E.coli cells and plated on (2xTY + 1% glucose + 100 µg/ml ampicillin) - 1.5% agar plates. Single colonies were grown in 2xTY + 0.1% glucose + 100 µg/ml ampicillin, and induced overnight at 30 degrees with 1 mM IPTG to achieve antibody expression. The resulting supematants were screened by ELISA using streptavidin-coated microtitre plates treated with 10 nM biotinylated-ED-B, and anti-FLAG M2 antibody (IBI Kodak, New Haven, CT) as detecting reagent. 32% of screened clones were positive in this assay and the three of them which gave the strongest ELISA signal (E1, A2 and G4) were sequenced and further characterised.

ELISA assays were performed using biotinylated ED-B recovered from a gel spot, biotinylated ED-B that had not been denatured, ED-B linked to adjacent fibronectin domains (recombinant protein containing the 7B89 domains), and a number of irrelevant antigens. Antibodies E1, A2 and G4 reacted strongly and specifically with all three ED-B containing proteins. This, together with the fact that the three recombinant antibodies could be purified from bacterial supematants using an ED-B affinity column, strongly suggests that they recognise an epitope present in the native conformation of ED-B. No reaction was detected with fibronectin fragments which did not contain the ED-B domain (data not shown).

In order to test whether the antibodies isolated against a gel spot had a good affinity towards the native antigen, real-time interaction analysis was performed using surface plasmon resonance on a BIAcore instrument as described (Neri et al. (1997) Nature Biotechnol., 15, 1271-1275). Monomeric fractions of E1, A2 and G4 scFv fragments bound to ED-B with affinity in the 107 - 108 M-1 range (Table 1).

As a further test of antibody specificity and usefulness, a 2D-PAGE immunoblot was performed, running on gel a lysate of the human melanoma cell line COLO-38, to which minute amounts of the ED-B containing recombinant 7B89 protein had been added (Figure 2). ScFv(E1) stained strongly and specifically only the 7B89 spot.

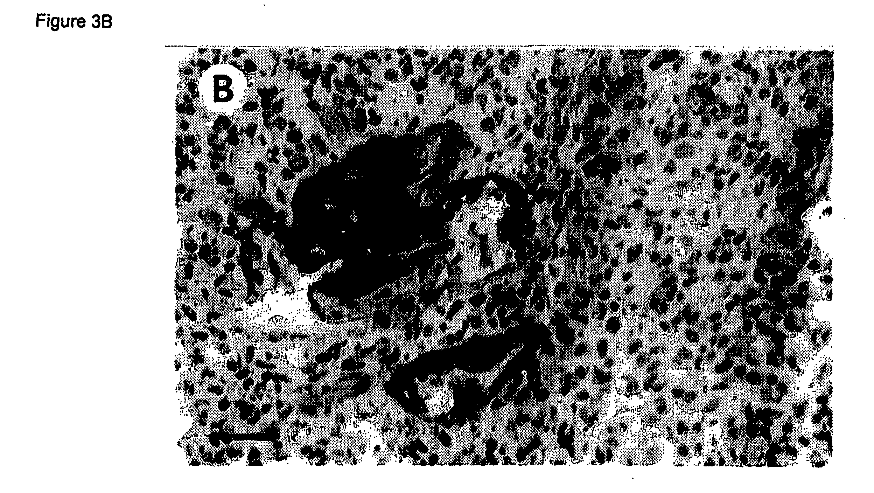

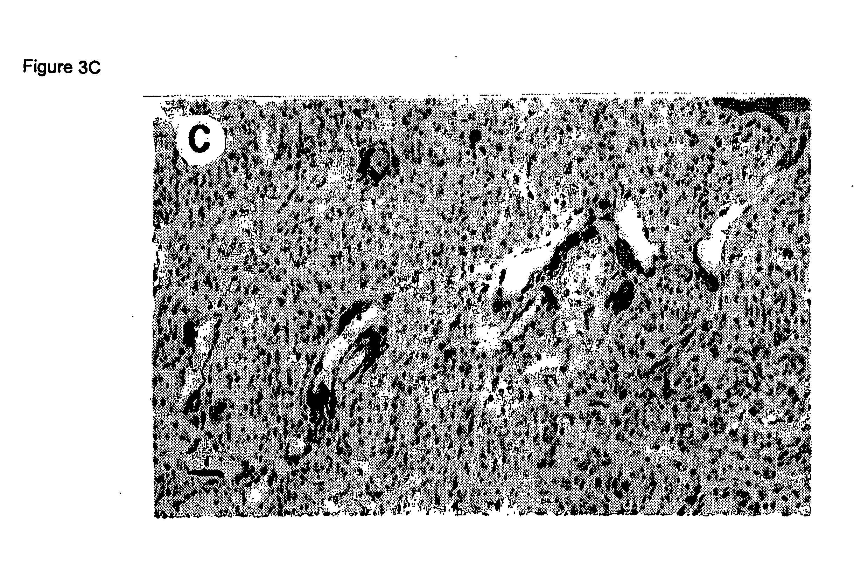

Antibodies E1, A2 and G4 were used to immunolocalise ED-B containing fibronectin (B-FN) in cryostat sections of glioblastoma multiforme, an aggressive human brain tumour with prominent angiogenetic processes. Figure 3 shows serial sections of glioblastoma multiforme, with the typical glomerulus-like vascular structures stained in red by the three antibodies. Immunostaining of sections of glioblastoma multiforme samples frozen in liquid nitrogen immediately after removal by surgical procedures, was performed as described (Carnemolla et al. (1996). Int. J. Cancer, 68, 397-405, Castellani et al. (1994). Int. J. Cancer, 59, 612-618). In short, immunostaining was performed using M2-anti-FLAG antibody (IBI Kodak), biotinylated anti-mouse polyclonal antibodies (Sigma), a streptavidin-biotin alkaline phosphatase complex staining kit (BioSpa, Milan, Italy) and naphtol-AS-MX-phosphate and fast-red TR (Sigma). Gill's hematoxylin was used as a counter-stain, followed by mounting in glycergel (Dako, Carpenteria, CA) as previously reported (Castellani et al. (1994). Int. J. Cancer, 59, 612-618).

Using similar techniques and the antibody L19 (see next example) we could also specifically stain new-forming blood vessels induced by implanting in the rabbit cornea polymer pellets soaked with angiogenic substances, such as vascular endothelial growth factor or phorbol esters.

### Example 2

### Isolation of a human scFv antibody fragment binding to the ED-B with sub-nanomolar affinity

ScFv(E1) was selected to test the possibility of improving its affinity with a limited number of mutations of CDR residues located at the periphery of the antigen binding site (Figure 1A). We combinatorially mutated residues 31-33, 50, 52 and 54 of the antibody VH, and displayed the corresponding repertoire on filamentous phage. These residues are found to frequently contact the antigen in the known 3D-structures of antibody-antigen complexes. The resulting repertoire of 4 x 10⁸ clones was selected for binding to the ED-B domain of fibronectin. After two rounds of panning, and screening of 96 individual clones, an antibody with 27-fold improved affinity was isolated (H10; Tables 1 and 2). Similarly to what others have observed with affinity-matured antibodies, the improved affinity was due to slower dissociation from the antigen, rather than by improved kon values (Schier et al. (1996). Gene, 169, 147-155, Ito (1995). J. Mol. Biol., 248, 729-732). The antibody light chain is often thought to contribute less to the antigen binding affinity as supported by the fact that both natural and artificial antibodies devoid of light chain can still bind to the antigen (Ward et al. (1989) Nature, 341, 544-546, Hamers-Casterman et al. (1993). Nature, 363, 446-448). For this reason we chose to randomise only two residues (32 and 50) of the VL domain, which are centrally located in the antigen binding site (Figure 1 a) and often found in 3D structures to contact the antigen. The resulting library, containing 400 clones, was displayed on phage and selected for antigen binding. From analysis of the dissociation profiles using real-time interaction analysis with a BIAcore instrument (Jonsson et al. (1991). BioTechniques, 11, 620-627) and koff measurements by competition experiments with electrochemiluminescent detection a clone (L19) was identified, that bound to the ED-B domain of fibronectin with a Kd = 54 pM (Tables 1 and 2).

Affinity maturation experiments were performed as follows. The gene of scFv(E1) was PCR amplified with primers LMB1bis (5'-GCG GCC CAG CCG GCC ATG GCC GAG-3') (SEQ ID NO:1) and DP47CDR1for (5'-GA GCC TGG CGG ACC CAG CTC ATM NNM NNM NNGCTA AAG GTG AAT CCA GAG GCT G-3') (SEQ ID NO:2) to introduce random mutations at positions 31-33 in the CDR1 of the VH (for numbering: 28), and with primers DP47CDR1back (5'-ATG AGC TGG GTC CGC CAG GCT CC-3') (SEQ ID NO:3) and DP47CDR2for (5'-GTC TGC GTA GTA TGT GGT ACC MNN ACT ACC MNN AAT MNN TGA GAC CCA CTC CAG CCC CTT-3') (SEQ ID NO:4) to randomly mutate positions 50,52,54 in CDR2 of the VH. The remaining fragment of the scFv gene, covering the 3'- portion of the VH gene, the peptide linker and the VL gene, was amplified with primers DP47CDR2back (5'-ACA TAC TAC GCA GAC TCC GTG AAG-3') (SEQ ID NO:5) and JforNot (5'-TCA TTC TCG ACT TGC GGC CGC TTT GAT TTC CAC CTT GGT CCC TTG GCC GAA CG-3') (SEQ ID NO:6) (94C 1 min, 60 C 1 min, 72 C 1 min). The three resulting PCR products were gel purified and assembled by PCR (21) with primers LMB1bis and JforNot (94°C 1 min, 60 C 1 min, 72 C 1 min). The resulting single PCR product was purified from the PCR mix, double digested with NotI/NcoI and ligated into NotI/NcoI digested pDN332 vector. Approximately 9 µg of vector and 3 µg of insert were used in the ligation mix, which was purified by phenolisation and ethanol precipitation, resuspended in 50 µl of sterile water and electroporated in electrocompetent TGI E.coli cells. The resulting affinity maturation library contained 4x10⁸ clones. Antibody-phage particles, produced as described (Nissim et al. (1994). EMBO J., 13, 692-698) were used for a first round of selection on 7B89 coated imunotube (Camemolia et al. (1996). Int. J. Cancer, 68, 397-405). The selected phages were used for a second round of panning performed with biotinylated ED-B, followed by capture with streptavidin coated magnetic beads (Dynal, Oslo. Norway; see previous paragraph). After selection, approximately 25% of the clones were positive in soluble ELISA (see previous chapter for experimental protocol). From the candidates positive in ELISA, we further identified the one (H10; Table 1) with lowest koff by BIAcore analysis (Jonsson et al. (1991), BioTechniques, 11, 620-627).

The gene of scFv(H10) was PCR amplified with primers LMB1bis and DPKCDR1for (5'-G TTT CTG CTG GTA CCA GGC TAA MNN GCT GCT GCT AAC ACT CTG ACT G) (SEQ ID NO:7) to introduce a random mutation at position 32 in CDR1 of the VL (for numbering: Chothia and Lesk (1987) J.MoLBiol., 196, 901-917), and with primers DPKCDR1back (5'-TTA GCC TGG TAC CAG CAG AAA CC-5') (SEQ ID NO:8) and DPKCDR2for (5'-GCC AGT GGC CCT GCT GGA TGC MNN ATA GAT GAG GAG CCT GGG AGC C-3') (SEQ ID NO:9) to introduce a random mutation at position 50 in CDR2 of the VL. The remaining portion of the scFv gene was amplified with oligos DPKCDR2back (5'-GCA TCC AGC AGG GCC ACT GGC-3') (SEQ ID NO:10) and JforNot (94C 1 min, 60 C 1 min, 72 C 1 min) The three resulting products were assembled, digested and cloned into pDN332 as described above for the mutagenesis of the heavy chain. The resulting library was incubated with biotinylated ED-B in 3% BSA for 30 min., followed by capture on a streptavidin-coated microtitre plate (Boehringer Mannheim GmbH, Germany) for 10 minutes. The phages were eluted with a 20 mM DTT solution (1,4-Dithio-DL-threitol, Fluka) and used to infect exponentially growing TG1 cells.

Analysis of ED-B binding of supematants from 96 colonies by ELISA and by BIAcore allowed the identification of clone L19. Anti-ED-B E1, G4, A2, H10 and L19 scFv antibody fragments selectively stain new-forming blood vessels in sections of aggressive tumours (Figure 3).

The above mentioned anti-ED-B antibody fragments were then produced inoculating a single fresh colony in 1 liter of 2xTY medium as previously described in Pini et al. ((1997), J. Immunol. Meth., 206, 171-182) and affinity purified onto a CNBr-activated sepharose column (Pharmacia, Uppsala, Sweden), which had been coupled with 10 mg of ED-B containing 7B89 recombinant protein (Camemolia et al. (1996). Int. J. Cancer, 68, 397-405). After loading, the column was washed with 50 ml of equilibration buffer (PBS, 1 mM EDTA, 0.5 M NaCl).

Antibody fragments were then eluted with triethylamine 100 mM, immediately neutralised with 1M Hepes, pH 7, and dialysed against PBS. Affinity measurements by BIAcore were performed with purified antibodies as described (Neri et al. (1997). Nature Biotechnol., 15 1271-1275) [Figure 4]. Band-shift analysis was performed as described (Neri et al. (1996). Nature Biotechnology, 14, 385-390), using recombinant ED-B fluorescently labeled at the N-terminal extremity (Camemolia et al. (1996). Int. J. Cancer, 68, 397-405, Neri et al. (1997).

Nature Biotechnol., 15 1271-1275) with the infrared fluorophore Cy5 (Amersham) [Figure 4]. BIAcore analysis does not always allow the accurate determination of kinetic parameters for slow dissociation reactions due to possible rebinding effects, baseline instability and long measurement times needed to ascertain that the dissociation phase follows a single exponential profile. We therefore performed measurements of the kinetic dissociation constant koff by competition experiments (Neri et al. (1996), Trends in Biotechnol., 14, 465-470) [Figure 4]. In brief, anti-ED-B antibodies (30 nM) were incubated with biotinylated ED-B (10 nM) for 10 minutes, in the presence of M2 anti-FLAG antibody (0,5 µg/ml) and polyclonal anti-mouse IgG (Sigma) which had previously been labeled with a rutenium complex as described (Deaver, D. R. (1995). Nature, 377, 758-760). To this solution, in parallel reactions, unbiotinylated ED-B (1 µM) was added at different times. Streptavidin-coated dynabeads, diluted in Origen Assay Buffer (Deaver, D.R. (1995). Nature, 377, 758-760) were then added (20 µl, 1 mg/ml), and the resulting mixtures analysed with a ORIGEN Analyzer (IGEN Inc. Gaithersburg, MD USA). This instrument detects an electrochemiluminescent signal (ECL) which correlates with the amount of scFv fragment still bound to the biotinylated ED-B at the end of the competition reaction. Plot of the ECL signal versus competition time yields a profile, that can be fitted with a single exponential with characteristic constant koff [Figure 4; Table 2].

### Example 3

### Targeting tumours with a high-affinity radiolabeled scFv specific for the ED-B domain of fibronectin

Radioiodinated scFv(L19) or scFv(D1.3) (an irrelevant antibody specific for hen egg lysozyme) were injected intravenously in mice with subcutaneously implanted murine F9 teratocarcinoma, a rapidly growing aggressive tumour. Antibody biodistributions were obtained at different time points (Figure 4). ScFv(L19) and scFv(D1.3) were affinity purified on an antigen column (Neri et al. (1997, Nature Biotechnol. 15, 1271-1273) and radiolabeled with iodine-125 using the lodogen method (Pierce, Rockford, IL, USA). Radiolabeled antibody fragments retained > 80% immunoreactivity, as evaluated by loading the radiolabeled antibody onto an antigen column, followed by radioactive counting of the flow-through and eluate fractions. Nude mice (12 weeks old Swiss nudes, males) with subcutaneously-implanted F9 murine teratocarcinoma (Neri et al. (1997) Nature Biotechnol. 15, 1271-1273) were injected with 3 µg (3-4 µCi) of scFv in 100 µl saline solution.

Tumour size was 50-250 mg, since larger tumours tend to have a necrotic centre. However, targeting experiments performed with larger tumours (300-600 mg) gave essentially the same results. Three animals were used for each time point. Mice were killed with humane methods, and organs weighed and radioactively counted. Targeting results of representative organs are expressed as percent of the injected dose of antibody per gram of tissue (% ID / g). ScFv(L19) is rapidly eliminated from blood through the kidneys; unlike conventional antibodies, it does not accumulate in the liver or other organs. Eight percent of the injected dose per gram of tissue localises on the tumour already three hours after injection; the subsequent decrease of this value is due to the fact that the tumour doubles in size in 24-48 hours. Tumour:blood ratios at 3, 5 and 24 hours after injection were 1.9, 3.9 and 11.8 respectively for L19, but always below 1.0 for the negative control antibody.

Radiolabeled scFv(L19) preferentially localises on tumours already few hours after injection, suggesting its usefulness for the immunoscintigraphic detection of angiogenesis in patients.

### Example 4

### Anti-ED-B antibodies selectively stain newly-formed ocular blood vessels

Angiogenesis, the formation of new blood vessels from pre-existing ones, is a characteristic process which underlies many diseases, including cancer and the majority of ocular disorders which result in loss of vision. The ability to selectively target and occlude neovasculature will open diagnostic and therapeutic opportunities.

We investigated whether B-FN is a specific marker of ocular angiogenesis and whether antibodies recognising B-FN could selectively target ocular neovascular structures in vivo upon systemic administration. To this aim we stimulated angiogenesis in the rabbit cornea, which allows the direct observation of new-blood vessels, by surgically implanting pellets containing vascular endothelial growth factor or a phorbol ester (Figure 7). Suaalfate (kind gift of Merck, Darmstadt, Germany) / hydron pellets containing either 800 ng vascular endothelial growth factor (Sigma) or 400 ng phorbol 12-myristate 13-acetate ("PMA"; Sigma) were implanted in the cornea of New Zealand White female rabbits as described [D'Amato, R.J., et al., *Proc. Natl. Acad. Sci. USA* 91, 4082-4085 (1994)]. Angiogenesis was induced by both factors. Rabbits were monitored daily. With both inducers newly formed blood vessels were strongly ED-B-positive in immunohistochemistry. For all further experiments, PMA pellets were used.

Immunohistochemical studies showed that L19 strongly stains the neovasculature induced in the rabbit cornea (Fig. 8; 9a), but not pre-existing blood vessels of the eye (Fig. 9b, c) and of other tissues (data not shown). Immunohistochemistry was performed as described [Camemolla, B. *et al., Int. J. Cancer* 68, 397-405 (1996)].

### Example 5

### The human antibody fragment L19, binding to the ED-B with sub-nanomolar affinity, targets ocular angiogenesis in vivo

Using the rabbit cornea model of angiogenesis described in the previous example, and an immunophotodetection methodology [Neri, D. *et al., Nature Biotechnol*. 15, 1271-1275 (1997)], we demonstrated that L19, chemically coupled to the red fluorophore Cy5, but not the antibody fragment (HyHEL-10)-Cy5 directed against an irrelevant antigen (Fig. 10a,b), selectively targets ocular angiogenesis upon intravenous injection. Fluorescent staining of growing ocular vessels was clearly detectable with L19 immediately after injection, and persisted for at least two days analogous to previous observations with tumour angiogenesis. Subsequent ex vivo immunofluorescent microscopic analysis on cornea sections confirmed the localisation of L19, but not of HyHEL-10, around vascular structures (Fig. 10c,d).

The demonstration of the antibody-based selective targeting of ocular neovascularisation, together with the reactivity of anti-B-FN antibodies in different species, warrants future clinical investigations. Immunofluorescence imaging could be useful for the early detection of ocular angiogenesis in risk patients, before lesions become manifest in fluoroangiography.

Some methodological details:

For ex vivo immunofluorescence and for some H/E stainings, corneas were fixed in 4% paraformaldehyde in PBS before embedding. Fluorescence photodetection experiments were performed with rabbits sedated using 5 mg/kg Acepromazin.

For targeting experiments, 3.5 mg of scFv(L19)₁-Cy5_{0.66} and 2.8 mg of scFv(HyHEL-10)₁-Cy5_{0.83} were injected intravenously in each rabbit (injection time = 15 min). A strong fluorescence in the corneal neovasculature was observed already immediately after injection of L19, but not of HyHEL-10, and persisted for several hours. As an additional test of specificity, rabbits injected the previous day with scFv(HyHEL-10)-Cy5 and negative in the fluorescence photodetection, were injected the next day with scFv(L19)-Cy5, and showed a strong fluorescent staining of corneal angiogenesis.

For fluorescence detection, the eye was illuminated with a tungsten halogen lamp (model Schott KL1500; Zeiss, Jena, Germany) equipped with a Cy5-excitation filter (Chroma, Brattleboro, VT, U.S.A.) and with two light guides whose extremities were placed at approximately 2 cm distance from the eye. Fluorescence was detected with a cooled C-5985 monochrome CCD-camera (Hamamatsu, Hamamatsu-City, Japan), equipped with C-mount Canon Zoom Lens (V6x16; 16-100 mm; 1: 1.9) and a 50 mm diameter Cy5 emission filter (Chroma), placed at 3-4 cm distance from the irradiated eye. Acquisition times were 0.4 s.

Cy5 fluoroangiography experiments were performed with the same experimental set up, but injecting intravenously 0.25 mg Cy5-Tris (the reaction product between Cy5-NHS and tris[hydroxymethyl]aminomethane; injection time = 5 s). Acquisition times were 0.2 s.

Antibody fragments were in scFv format. The purification of scFv(L19) and scFv(HyHEL-10) and their labeling with the N-hydroxysuccinimide (NHS) esters of indocyanine dyes have been described elsewhere [Neri, D. *et al., Nature Biotechnol.* 15, 1271-1275 (1997); Fattorusso, R., et al. (1999) *Structure,* 7, 381-390]. Antibody : Cy5 labeling ratios for the two antibodies were 1.5 : 1 and 1.2 : 1, respectively. Cy5-NHS was purchased from Amersham Pharmacia Biotech (Zurich, Switzerland), ovalbumin from Sigma (Buchs, Switzerland).

After the labeling reaction, antibody conjugates were separated from unincorporated fluorophore or photosensitiser using PD-10 columns (Amersham Pharmacia Biotech) equilibrated in 50 mM phosphate, pH 7.4, 100 mM NaCl (PBS). Immunoreactivity of antibody conjugates was measured by affinity chromatography on antigen columns [Neri, D. *et al., Nature Biotechnol*. 15, 1271-1275 (1997)] and was in all cases > 78%. Immunoconjugates were analysed by sodium dodecyl sulfate polyacrylamide gel electrophoresis and migrated as a band of MW = 30'000 Dalton (purity ³ 90%).

### Example 6

### The human antibody fragment L19 chemically conjugated to the photosensitiser Sn (IV) chlorine e6, selectively targets ocular angiogenesis and mediates its occlusion upon irradiation with red light

To test whether selective vessel ablation could be achieved by virtue of the antibody-mediated targeting, we injected rabbits with the L19 antibody fragment or an irrelevant protein that does not localise in newly formed blood vessels (ovalbumin) coupled to the photosensitiser tin (IV) chlorin e₆ (hereafter named "PS"). The eyes of injected animals were irradiated with red light (light dose = 78 J/cm²). Representative results are depicted in Figure 11. A striking macroscopic difference was observed 16 h after irradiation in rabbits treated with L19-PS (Fig. 11 a,b), with coagulation of the corneal neovasculature but not of vessels in the conjunctiva or in other ocular structures. Fluoroangiography with the indocyanine fluorophore Cy5 (Fig. 11c) confirmed vessel occlusion as a characteristic hypofluorescent area. On the contrary, hyperfluorescent areas were observed in the leaky neovasculature of non-irradiated eyes (Fig. 11d,h). No macroscopic alteration was detectable in the irradiated vessels of rabbits treated with ovalbumin-PS (Fig. 11e-g), either ophthalmoscopically or by Cy5 fluoroangiography. The effect of irradiation of the targeted L19-PS conjugate at early stages of corneal angiogenesis are shown in Fig. 11i-l. Selectively coagulated blood vessels were macroscopically visible in live animals (Fig. 11i,j) and even more evident in animals immediately after euthanasia (Fig. 11 k,l).

Photodynamic damage was further investigated using microscopic techniques. After irradiation, vessel occlusion could be detected by standard hematoxylin/eosin (H/E) staining techniques in both non-fixed and paraformaldehyde-fixed cornea sections of animals treated with L19-PS (Fig. 11 b,f,j), but not of those treated with ovalbumin-PS (Fig. 11 a,e,i). Apoptosis in the portion of the cornea targeted by the photosensitiser conjugate was clearly visible in the fluorescent TUNEL assay (Fig. 11c,g), but hardly detectable ink negative controls (Fig. 11d,h). A higher magnification view showed apoptosis of endothelial cells in vascular structures (Fig. 11g). No damage to blood vessels of the iris, sclera and conjunctiva of treated animals could be observed either by TUNEL assay (not shown) or by H/E staining (Fig. 11k,l).

Selective photodynamic ablation of neovasculature promises to be beneficial for the treatment of ocular disorders and of other angiogenesis-related pathologies that are accessible to irradiation using light diffusers or fibre optic techniques. The results of this study clearly demonstrate that ocular neovasculature can be selectively occluded without damaging pre-existing blood vessels and normal tissues.

Some methodological details:

Tin (IV) chlorin e₆ was selected from a panel of photosensitisers, on the basis of their potency, solubility and specificity, after coupling to a rabbit anti-mouse polyclonal antibody (Sigma). These immunoconjugates were screened by targeted photolysis of red blood cells coated with a monoclonal antibody specific for human CD47 (#313441A; Pharmingen, San Diego CA, U.S.A.). Tin (IV) chlorin e₆ was prepared as described [Lu, X.M. *et al., J. Immunol. Methods* 156, 85-99 (1992)]. For coupling to proteins, tin (IV) chlorin e₆ (2 mg/ml) was mixed for 30 min at room temperature in dimethylformamide with a ten-fold molar excess of EDC (N'-3-dimethylaminopropyl-N-ethylcarbodiimide hydrochloride, Sigma) and NHS (N-hydoxysuccinimide, Sigma). The resulting activated mixture was then added to an eight-fold larger volume of protein solution (1 mg/ml) and incubated at room temperature for 1h.

After the labeling reaction, antibody conjugates were separated from unincorporated fluorophore or photosensitiser using PD-10 columns (Amersham Pharmacia Biotech) equilibrated in 50 mM phosphate, pH 7.4, 100 mM NaCl (PBS). Immunoreactivity of antibody conjugates was measured as described in the previous Example.

For photokilling experiments, rabbits were injected intravenously with 12 mg scFv(L19)₁ -tin (IV) chlorin e6_{0.8} or 38 mg ovalbumin₁ - tin (IV) chlorin e6_{0.36}, and kept in the dark for the duration of the experiment. Eight hours after injection, rabbits were anesthesised with ketamin (35 mg/kg) / xylazine (5 mg/kg) /acepromazin (1 mg/kg), and one of the two eyes was irradiated for 13 min with a Schott KL1500 tungsten halogen lamp equipped with a Cy5 filter (Chroma) and with two light guides whose extremities were placed at 1 cm distance from the eye. The illuminated area was approximately 1 cm², with an irradiation power density of 100 mW/cm², measured using a SL818 photodetector (Newport Corp., Irvine, CA, U.S.A.). No sign of animal discomfort after irradiation was observed. As a preventive measure, rabbits received analgesics after irradiation (buprenorphine 0.03 mg/Kg). To monitor photokilling, eyes were investigated with an ophthalmoscope and photographed using a fundus camera KOWA SL-14 (GMP SA, Rennens, Lausanne, Switzerland). Five rabbits were treated with each of the tin (IV) chlorin e₆ conjugates and irradiated in one eye only, the other eye serving as an internal negative control. As additional control, two rabbits were irradiated only, but received no photosensitiser conjugate.

Immediately after rabbits' euthanasia with an overdose of anaesthetic, eyes were enucleated, corneas removed, then embedded in Tissue Tek (Sakura Finetechnical, Tokyo, Japan) and frozen. For ex vivo immunofluorescence and for some H/E stainings, corneas were fixed in 4% paraformaldehyde in PBS before embedding. Cryostat sections of 5 µm were used for further microscopic analysis.

Fluorescent TUNEL assays were performed according to manufacturer's instructions (Roche Diagnostic, Rotkreuz, Switzerland).

**Table 1:**

| Sequences of selected anti-ED-B antibody clones | | | | | | |
|---|---|---|---|---|---|---|
| | VH chain | | | VL chain | | |
| Clone | 31-33* | 50-54* | 95-98* | 32* | 50* | 91-96* |
| A2 | SYA | AISGSG | GLSI | Y | G | NGWYPW |
| | | | | | | |
| G4 | SYA | AISGSG | SFSF | Y | G | GGWLPY |
| | | | | | | |
| E1 | SYA | AISGSG | FPFY | Y | G | TGRIPP |
| | | | | | | |
| H10 | SFS | SIRGSS | FPFY | Y | G | TGRIPP |
| | | | | | | |
| L19 | SFS | SIRGSS | FPFY | Y | Y | TGRIPP |

| | | | | | | |
|---|---|---|---|---|---|---|
| Relevant amino acid positions (*: numbering according to Tomlinson et al. (1995) EMBO J., 14, 4628-4638) of antibody clones isolated from the designed synthetic libraries. Single amino acid codes are used according to standard IUPAC nomenclature. | | | | | | |

**Table 2:**

| Affinities of anti-ED-B scFv fragments | | | | |
|---|---|---|---|---|
| Clone | kon (s⁻¹M⁻¹) | koff (s⁻¹)^{B} | koff (s⁻¹)^{C} | K_{d}(M)* |
| | | | | |
| A2 | 1.5 x 10⁵ | 2.8 x 10⁻³ | - | 1.9 x 10⁻⁸ |
| | | | | |
| G4 | 4.0 x 10⁴ | 3.5 x 10⁻³ | - | 8.7 x 10⁻⁸ |
| | | | | |
| E1 | 1.6 x 10⁵ | 6.5 x 10⁻³ | - | 4.1 x 10⁻⁸ |
| | | | | |
| H10 | 6.7 x 10⁴ | 5.6 x 10⁻⁴ | 9.9 x 10⁻⁵ | 1.5 x 10⁻⁹ |
| | | | | |
| L19 | 1.1 x 10⁵ | 9.6 x 10⁻⁵ | 6.0 x 10⁻⁶ | 5.4 x 10⁻¹¹ |

| | | | | |
|---|---|---|---|---|
| *) K_{d} = k_{off} / kₒₙ. For the high-affinity binders H10 and L19, k_{off} values from BIAcore experiments are not sufficietly reliable due to effects of the negatively-charged carboxylated solid dextran matrix; Kd values are therefore calculated from k_{off} measurements obtained by competition experiments (Experimental Procedures). k_{off}, kinetic dissociation constant; kₒₙ, kinetic association constant; K_{d}, dissociation constant. B = measured on the BIAcore; C = measured by competition with electrochemiluminescent detection. Values are accurate to +/- 50%, on the basis of the precision of concentration determinations. | | | | |

### SEQUENCE LISTING

<110> EIDGENOESSISCHE TECHNISCHE HOCHSCHULE ZURICH
<120> Specific binding molecules for scintigraphy, conjugates containing them and therapeutic method for treatment of angiogenesis
<130> 1900PTEP
<140>
   <141>
<150> US 09/075,338
   <151> 1998-05-11
   <150> US
   <151> 1999-04-28
<160> 21
<170> Patent In Ver. 2.0
<210> 1
   <211> 24
   <212> DNA
   <213> Artificial Sequence
<220>
   <223> Description of Artificial Sequence: PCR primer: LMB1bis
<400> 1
   gcggcccagc cggccatggc cgag 24
<210> 2
   <211> 54
   <212> DNA
   <213> Artificial Sequence
<220>
   <223> Description of Artificial Sequence: PCR primer: DP47CDR1for
<400> 2
   gagcctggcg gacccagctc atmnnmnnmn ngctaaaggt gaatccagag gctg 54
<210> 3
   <211> 23
   <212> DNA
   <213> Artificial Sequence
<220>
   <223> Description of Artificial Sequence: PCR primer: DP47CDR1back
<400> 3
   atgagctggg tccgccaggc tcc 23
<210> 4
   <211> 60
   <212> DNA
   <213> Artificial Sequence
<220>
   <223> Description of Artificial Sequence: PCR primer: DP47CDR2for
<400> 4
   gtctgcgtag tatgtggtac cmnnactacc mnnaatmnnt gagacccact ccagcccctt 60
<210> 5
   <211> 24
   <212> DNA
   <213> Artificial Sequence
<220>
   <223> Description of Artificial Sequence: PCR primer: DP47CDR2back
<400> 5
   acatactacg cagactccgt gaag 24
<210> 6
   <211> 53
   <212> DNA
   <213> Artificial Sequence
<220>
   <223> Description of Artificial Sequence: PCR primer: JforNot
<400> 6
   tcattctcga cttgcggccg ctttgatttc caccttggtc ccttggccga acg 53
<210> 7
   <211> 47
   <212> DNA
   <213> Artificial Sequence
<220>
   <223> Description of Artificial Sequence: PCR primer: DPKCDR1for
<400> 7
   gtttctgctg gtaccaggct aamnngctgc tgctaacact ctgactg 47
<210> 8
   <211> 23
   <212> DNA
   <213> Artificial Sequence
<220>
   <223> Description of Artificial Sequence: PCR primer: DPKCDR1back
<400> 8
   ttagcctggt accagcagaa acc 23
<210> 9
   <211> 46
   <212> DNA
   <213> Artificial Sequence
<220>
   <223> Description of Artificial Sequence: PCR primer: DPKCDR2for
<400> 9
   gccagtggcc ctgctggatg cmnnatagat gaggagcctg ggagcc 46
<210> 10
   <211> 21
   <212> DNA
   <213> Artificial Sequence
<220>
   <223> Description of Artificial Sequence: PCR primer: DPKCDR2back
<400> 10
   gcatccagca gggccactgg c 21
<210> 11
   <211> 45
   <212> DNA
   <213> Artificial Sequence
<220>
   <223> Description of Artificial Sequence: PCR primer: DP47baNco
<400> 11
   gcggcccagc atgccatggc cgaggtgcag ctgttggagt ctggg 45
<210> 12
   <211> 55
   <212> DNA
   <213> Artificial Sequence
<220>
   <223> Description of Artificial Sequence: PCR primer: CDR3for
<400> 12
   ggttccctgg ccccagtagt caaamnnmnn mnnmnntttc gcacagtaat atacg 55
<210> 13
   <211> 24
   <212> DNA
   <213> Artificial Sequence
<220>
   <223> Description of Artificial Sequence: PCR primer: VHpullth
<400> 13
   gcggcccagc atgccatggc cgag 24
<210> 14
   <211> 66
   <212> DNA
   <213> Artificial Sequence
<220>
   <223> Description of Artificial Sequence: PCR primer: Jassm
<400> 14
   cccgctaccg ccactggacc catcgccact cgagacggtg accagggttc cctggcccca 60
   gtagtc 66
<210> 15
   <211> 62
   <212> DNA
   <213> Artificial Sequence
<220>
   <223> Description of Artificial Sequence: PCR primer: DPK22assm
<400> 15
   gatgggtcca gtggcggtag cgggggcgcg tcgactggcg aaattgtgtt gacgcagtct 60
   cc 62
<210> 16
   <211> 63
   <212> DNA
   <213> Artificial Sequence
<220>
   <223> Description of Artificial Sequence: PCR primer: DPK3for
<400> 16
   caccttggtc ccttggccga acgtmnncgg mnnmnnaccm nnctgctgac agtaatacac 60
   tgc 63 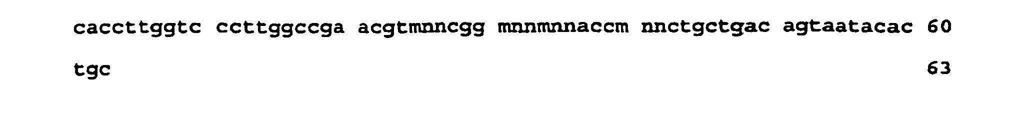
<210> 17
   <211> 56
   <212> DNA
   <213> Artificial Sequence
<220>
   <223> Description of Artificial Sequence: PCR primer: Jfornot
<400> 17
   gagtcattct cgacttgcgg ccgctttgat ttccaccttg gtcccttggc cgaacg 56
<210> 18
   <211> 24
   <212> DNA
   <213> Artificial Sequence
<220>
   <223> Description of Artificial Sequence: PCR primer: VLpullth
<400> 18
   gatgggtcca gtggcggtag cggg 24
<210> 19
   <211> 116
   <212> PRT
   <213> VH antibody specific for ED-B domain of fibronectin
<400> 19
<210> 20
   <211> 14
   <212> PRT
   <213> antibody linker
<400> 20
   Gly Asp Gly Ser Ser Gly Gly Ser Gly Gly Ala Ser Thr Gly
   1 5 10
<210> 21
   <211> 108
   <212> PRT
   <213> VL antibody specific for ED-B domain of fibronectin
<400> 21
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Tyr Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Thr Gly Arg Ile Pro
   85 90 95
   Pro Thr Phe Gly Gln Gly Thr Lys Val Glu Ile Lys
   100 105

## Claims

1. An isolated antibody with specific affinity for a characteristic epitope of the Extra Domain B (ED-B) domain of fibronectin, wherein the antibody has an affinity to said ED-B epitope in the subnanomolar range and recognizes ED-B(+) fibronectin.

2. The antibody according to claim 1 wherein the antibody binds the ED-B domain of fibronectin with a Kd of 54 pM.

3. The antibody according to claim 1, with the following amino acid sequence:

4. The antibody according to claim 1, wherein the antibody is a functionally equivalent variant form of L19, wherein L19 has the amino acid sequence of SEQ ID NOs: 19, 20 and 21.

5. The antibody according to any one of claims 1 to 4, wherein said antibody is in the scFv format.

6. The antibody according to any one of claims 1 to 4, wherein said antibody is a whole antibody.

7. The antibody according to any one of claims 1 to 6, wherein the antibody is radiolabeled.

8. The antibody according to any one of claims 1 to 6, wherein the antibody is radioiodinated.

9. An antibody according to any one of claims 1 to 8 for use in a method of rapid targeting markers of angiogenesis.

10. The antibody as claimed in claim 9 for immunoscintigraphic detection of angiogenesis.

11. The antibody as claimed in claim 10 for detecting diseases **characterized by** vascular proliferation such as diabetic retinopathy, age-related macular degeneration or tumours.

12. A diagnostic kit comprising the antibody according to any one of claims 1 to 8 and one or more reagents necessary for detecting angiogenesis.

13. Use of the antibody according to any one of claims 1 to 8 in the manufacture of a diagnostic agent for diagnosis of tumours or a disease **characterized by** vascular proliferation.

14. Use of the antibody according to any one of claims 1 to 8 in the manufacture of a medicament for therapy of tumours or a disease **characterized by** vascular proliferation.

15. A conjugate comprising an antibody according to any one of claims 1 to 8 and a molecule capable of inducing blood coagulation and blood vessel occlusion.

16. A conjugate according to claim 15 wherein the molecule capable of inducing blood coagulation and blood vessel occlusion is a photoactive molecule.

17. A conjugate according to claim 16 wherein the photoactive molecule is a photosensitizer.

18. A conjugate according to claim 17 wherein the photosensitizer absorbs at a wavelength above 600nm.

19. A conjugate according to claim 18 wherein the photsensitizer is a derivative of tin (IV) chlorine e6.

20. A conjugate according to any one of claims 15 to 19 for use in therapeutic treatment of a tumour or disease **characterised by** vascular proliferation in the human or animal body.

21. Use of a conjugate according to any one of claims 15 to 19 for the preparation of an injectable composition for the treatment of an angiogenesis-related pathology.

22. Use according to claim 21, wherein the angiogenesis-related pathology is caused by or associated with ocular angiogenesis.

## Patentansprüche

1. Isolierter Antikörper mit spezifischer Affinität für ein charakteristisches Epitop der Extra-Domain-B- (ED-B-) Domäne von Fibronectin, worin der Antikörper eine Affinität für dieses ED-B-Epitop im subnanomolaren Bereich aufweist und ED-B(+)-Fibronectin erkennt.

2. Antikörper nach Anspruch 1, worin der Antikörper die ED-B-Domäne von Fibronectin mit einer Kd von 54 pM bindet.

3. Antikörper nach Anspruch 1 mit der folgenden Aminosäuresequenz:

4. Antikörper nach Anspruch 1, worin der Antikörper eine funktionell äquivalente Variante von L19 ist, worin L19 die Aminosäuresequenz der Seq.-ID Nr. 19, 20 und 21 aufweist.

5. Antikörper nach einem der Ansprüche 1 bis 4, worin der Antikörper im scFv-Format vorliegt.

6. Antikörper nach einem der Ansprüche 1 bis 4, worin der Antikörper ein vollständiger Antikörper ist.

7. Antikörper nach einem der Ansprüche 1 bis 6, worin der Antikörper radioaktiv markiert ist.

8. Antikörper nach einem der Ansprüche 1 bis 6, worin der Antikörper mit radioaktivem lod markiert ist.

9. Antikörper nach einem der Ansprüche 1 bis 8 zur Verwendung in einem Verfahren zum raschen Targeting von Angiogenese-Markern.

10. Antikörper nach Anspruch 9 zur immunszintigraphischen Detektion von Angiogenese.

11. Antikörper nach Anspruch 10 zur Detektion von Erkrankungen, die durch vaskuläre Proliferation **gekennzeichnet** sind, wie beispielsweise Retinopathia diabetica, altersbedingte Makuladegeneration oder Tumoren.

12. Diagnoseset, umfassend einen Antikörper nach einem der Ansprüche 1 bis 8 und ein oder mehrere zur Detektion von Angiogenese erforderliche Reagenzien.

13. Verwendung des Antikörpers nach einem der Ansprüche 1 bis 8 zur Herstellung eines Diagnosemittels zur Diagnose von Tumoren oder einer Erkrankung, die durch vaskuläre Proliferation **gekennzeichnet** ist.

14. Verwendung des Antikörpers nach einem der Ansprüche 1 bis 8 zur Herstellung eines Medikaments zur Therapie von Tumoren oder einer Erkrankung, die durch vaskuläre Proliferation **gekennzeichnet** ist.

15. Konjugat, umfassend einen Antikörper nach einem der Ansprüche 1 bis 8 und ein Molekül, das in der Lage ist, Blutkoagulation und Blutgefäßokklusion zu induzieren.

16. Konjugat nach Anspruch 15, worin das Molekül, das in der Lage ist, Blutkoagulation und Blutgefäßokklusion zu induzieren, ein photoaktives Molekül ist.

17. Konjugat nach Anspruch 16, worin das photoaktive Molekül ein Photosensibilisator ist.

18. Konjugat nach Anspruch 17, worin der Photosensibilisator bei einer Wellenlänge oberhalb von 600 nm absorbiert.

19. Konjugat nach Anspruch 18, worin der Photosensibilisator ein Derivat von Zinn(IV)-Chlorin e6 ist.

20. Konjugat nach einem der Ansprüche 15 bis 19 zur Verwendung bei der therapeutischen Behandlung eines Tumors oder einer Erkrankung, die durch vaskuläre Proliferation **gekennzeichnet** ist, im menschlichen Körper oder Tierkörper.

21. Verwendung eines Konjugats nach einem der Ansprüche 15 bis 19 zur Herstellung einer injizierbaren Zusammensetzung zur Behandlung einer mit Angiogenese verbundenen Pathologie.

22. Verwendung nach Anspruch 21, worin die mit Angiogenese verbundene Pathologie durch okulare Angiogenese verursacht wird oder damit in Verbindung steht.

## Revendications

1. Anticorps isolé avec une affinité spécifique pour un épitope caractéristique du domaine de fibronectine Extra Domaine B (ED-B), où l'anticorps a une affinité pour ledit épitope de ED-B dans la gamme subnanomolaire et
reconnaît la fibronectine ED-B(+)

2. Anticorps selon la revendication 1, où l'anticorps se lie au domaine ED-B de la fibronectine avec une valeur de Kd de 54 pM.

3. Anticorps selon la revendication 1, ayant la séquence d'acides aminés qui suit :

4. Anticorps selon la revendication 1, où l'anticorps est une forme variante fonctionnellement équivalente de L19, où L19 a la séquence d'acides aminés de SEQ ID NOs : 19, 20 et 21.

5. Anticorps selon l'une quelconque des revendications 1 à 4, où ledit anticorps est sous le format scFv.

6. Anticorps selon l'une quelconque des revendications 1 à 4, où ledit anticorps est un anticorps entier.

7. Anticorps selon l'une quelconque des revendications 1 à 6, où l'anticorps est radiomarqué.

8. Anticorps selon l'une quelconque des revendications 1 à 6, où l'anticorps est radio-iodé.

9. Anticorps selon l'une quelconque des revendications 1 à 8, à utiliser dans une méthode de ciblage rapide des marqueurs d'angiogénèse.

10. Anticorps selon la revendication 9 pour une détection immunoscintigraphique de l'angiogénèse.

11. Anticorps selon la revendication 10 pour la détection de maladies **caractérisées par** une prolifération vasculaire comme la rétinopathie diabétique, la dégénérescence maculaire en rapport avec l'âge ou les tumeurs.

12. Kit de diagnostic comprenant l'anticorps selon l'une quelconque des revendications 1 à 8 et un ou plusieurs réactifs nécessaires pour détecter l'angiogénèse.

13. Utilisation de l'anticorps selon l'une quelconque des revendications 1 à 8 dans la fabrication d'un agent de diagnostic pour le diagnostic des tumeurs ou d'une maladie **caractérisée par** une prolifération vasculaire.

14. Utilisation de l'anticorps selon l'une quelconque des revendications 1 à 8 dans la fabrication d'un médicament pour la thérapie des tumeurs ou d'une maladie **caractérisée par** une prolifération vasculaire.

15. Conjugué comprenant un anticorps selon l'une quelconque des revendications 1 à 8 et une molécule capable d'induire la coagulation du sang et l'occlusion d'un vaisseau sanguin.

16. Conjugué selon la revendication 15 où la molécule capable d'induire la coagulation du sang et l'occlusion d'un vaisseau sanguin est une molécule photoactive.

17. Conjugué selon la revendication 16 où la molécule photoactive est un photosensibilisateur.

18. Conjugué selon la revendication 17 où le photosensibilisateur absorbe à une longueur d'onde au-dessus de 600 nm.

19. Conjugué selon la revendication 18 où le photosensibilisateur est un dérivé d'étain (IV) chlore e6.

20. Conjugué selon l'une quelconque des revendications 15 à 19 à utiliser dans le traitement thérapeutique d'une tumeur ou maladie **caractérisée par** une prolifération vasculaire dans le corps humain ou animal.

21. Utilisation d'un conjugué selon l'une quelconque des revendications 15 à 19 pour la préparation d'une composition injectable pour le traitement d'une pathologie en rapport avec l'angiogénèse.

22. Utilisation selon la revendication 21, où la pathologie en rapport avec l'angiogénèse est provoquée par ou associée à l'angiogénèse oculaire.
